(19) **Europäisches Patentamt**
**European Patent Office**
**Office européen des brevets**

(11) **EP 2 587 998 B1**

(12) **EUROPEAN PATENT SPECIFICATION**

(45) Date of publication and mention
of the grant of the patent:
**19.11.2014 Bulletin 2014/47**

(51) Int Cl.:
***A61B 5/145*** *(2006.01)* ***G06F 19/00*** *(2011.01)*

(21) Application number: **11729447.0**

(86) International application number:
**PCT/GB2011/000992**

(22) Date of filing: **30.06.2011**

(87) International publication number:
**WO 2012/001365 (05.01.2012 Gazette 2012/01)**

(54) **METHODS TO ENSURE STATISTICAL POWER FOR AVERAGE PRE AND POST-PRANDIAL GLUCOSE DIFFERENCE MESSAGING**

VERFAHREN ZUR SICHERSTELLUNG VON STATISTISCHER AUSSAGEKRAFT FÜR DIE MESSUNG DER DURCHSCHNITTLICHEN PRÄ- UND POSTPRANDIALEN GLUKOSEDIFFERENZ

PROCÉDÉ ASSURANT UNE PUISSANCE STATISTIQUE POUR L'ENVOI DE MESSAGES RELATIFS À LA DIFFÉRENCE ENTRE LA GLYCÉMIE À JEUN ET LA GLYCÉMIE POSTPRANDIALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2010 US 360137 P**

(43) Date of publication of application:
**08.05.2013 Bulletin 2013/19**

(73) Proprietor: **Lifescan Scotland Limited
Inverness IV2 3ED (GB)**

(72) Inventors:
• **BLYTHE, Stephen, Patrick
Inverness IV2 3ED (GB)**
• **MALECHA, Michael
Muir of Ord IV6 7TL (GB)**

(74) Representative: **Brunner, John Michael Owen
Carpmaels & Ransford LLP
One Southampton Row
London WC1B 5HA (GB)**

(56) References cited:
**WO-A1-97/18464** **US-A1- 2006 025 931**
**US-A1- 2009 240 127**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

The incidence of diabetes is currently exploding worldwide. It is estimated that more than 44 million people in the United States alone are pre-diabetic, and unaware they have the condition. Diabetes results in a loss of control of blood sugar concentration. Complications from diabetes through loss of blood sugar control and in particular high blood sugars (hyperglycemia) can be debilitating and even life threatening. Health costs for treating such complications can be significant.

A groundbreaking study 'The Diabetes Control and Complications Trial' (DCCT) carried out over 9 years (1984 - 1993) and involving 1441 people with insulin-dependent diabetes throughout the USA and Canada, compared the effects of intensive and conventional insulin treatments on the development and progression of diabetic complications. Diabetics can be at risk of conditions associated with microvascular disease that can lead to cardiovascular disease, retinopathy (eye disease), neuropathy (nerve damage) and nephropathy (kidney disease). Other conditions associated with diabetes include circulatory problems, heart attacks and strokes.

Results from the DCCT study showed the lowest incidence of complications were found amongst those patients receiving intensive treatment (those having blood glucose levels averaging 8.6 mmol/l and glycated hemoglobin (HbA1c) levels of around 7%), compared to those in the conventional treatment group. HbA1c is a long term indicator of a patient's average blood sugar concentration, or long term glycemic condition typically over the previous two or three months. The DCCT and other similar studies have repeatedly demonstrated that the most effective way to prevent long-term diabetes-related complications is by strict control of blood glucose levels. One technique to monitor blood glucose level and hence HbAlc level is to measure blood glucose using commercially available glucose test strips.

US2009/0240127 A1 discloses a method of alerting a user with a diabetes management device that the user's blood glucose data around a meal event has exceeded a predefined threshold value.

Electrochemical glucose test strips, such as those used in the OneTouch® Ultra® whole blood testing kit, which is available from LifeScan, Inc., are designed to measure the concentration of glucose in a blood sample from patients with diabetes. The measurement of glucose is based upon the specific oxidation of glucose by the enzyme glucose oxidase (GO), where the current generated is proportional to the glucose content of the sample. As it can be very important to know the concentration of glucose in blood, particularly in people with diabetes, test meters have been developed to enable the average person to sample and test their blood for determining their glucose concentration at any given time. The current generated is detected by the test meter and converted into a glucose concentration reading using an algorithm that relates the test current to a glucose concentration via a simple mathematical formula. In general, such test meters work in conjunction with a disposable test strip that typically includes a sample receiving chamber and at least two electrodes disposed within the sample-receiving chamber, in addition to an enzyme (e.g. glucose oxidase) and a mediator (e.g. ferricyanide). In use, the patient pricks their finger or other convenient site to induce bleeding and introduces a blood sample to the sample-receiving chamber, thus starting the chemical reaction.

People with diabetes often rely upon the use of a blood glucose meter in conjunction with advice from their physicians for managing their disease. In addition, people with diabetes often use a logbook to keep track of their glucose concentration measurements. Under certain circumstances, interpreting a large number of glucose concentration measurements in a logbook format can be difficult, complex, and time consuming. To further complicate matters, physicians usually have limited time constraints in assisting people with diabetes to interpret a large number of glucose concentration measurements. Furthermore, the assessment is often further compounded by the physicians need to assess the effect of insulin or type of insulin on the patient, as well as other physiological parameters or external parameters. An additional hurdle for physicians or clinicians is the time constraint placed upon an office visit for the patient due to the economics of running a medical office. It is believed that in most cases, a physician or clinician typically spends less than approximately seven (7) minutes per patient, which results in little or no time for assessment or guidance for the patient. There is therefore a need for a measurement system that provides for easy and quick assessment of glycemic data.

**SUMMARY OF THE DISCLOSURE**

A method of alerting a user with the diabetes management device that the user's blood glucose data around a meal event has exceeded a predetermined differential threshold is provided as defined in claim 1.

**BRIEF DESCRIPTION OF THE DRAWINGS**

The accompanying drawings, which are incorporated herein and constitute part of this specification, illustrate presently preferred embodiments of the invention, and, together with the general description given above and the detailed description given below, serve to explain features of the invention (wherein like numerals represent like elements). A detailed understanding of the features and advantages of the present invention will be obtained by reference to the following detailed description that sets forth illustrative embodiments, in which the principles of the invention are utilized, and the accompanying drawings of which:

Figure 1A illustrates a diabetes management system that includes an analyte measurement and data management unit and data communication devices;

Figure 1B illustrates, in simplified schematic, an exemplary circuit board of a diabetes data management unit;

Figure 2A illustrates a size of samples versus confidence interval for statistical analysis;

Figure 2B shows a method to determine a calculated number of sample m that provides sufficient certainty in the accuracy of data needed for further analysis;

Figure 3A shows a method to determine whether a differential number of pre and post prandial glucose measurements for a particular meal has exceeded a preset threshold sufficient to warrant an alert to the user;

Figure 3B shows an alternative method which is less calculation intensive in determining whether a differential number of pre and post prandial glucose measurements for a particular meal has exceeded a preset threshold sufficient to warrant an alert to the user;

Figure 4 shows an example plot of the true function for $Q_{\alpha,N}$ compared against the simple approximation for a critical value $t_{\alpha,N-1}$,; and

Figure 5 shows an example embodiment of a message displayed via a portable handheld diabetes management unit to a patient.

## MODES OF CARRYING THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient represents a preferred embodiment.

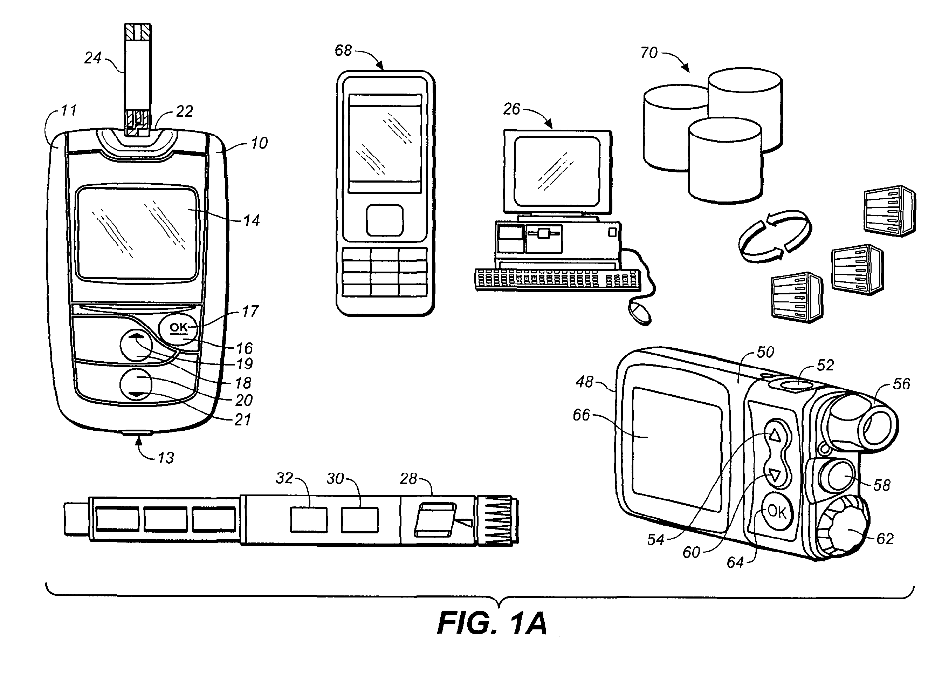

Figure 1A illustrates a diabetes management system that includes an analyte measurement and management unit 10, therapeutic dosing devices (28 or 48), and data/communication devices (68, 26, or 70). Analyte measurement and management unit 10 can be configured to wirelessly communicate with a handheld glucose-insulin data management unit or DMU such as, for example, an insulin pen 28, an insulin pump 48, a mobile phone 68, or through a combination of the exemplary handheld glucose-insulin data management unit devices in communication with a personal computer 26 or network server 70, as described herein. As used herein, the nomenclature "DMU" represents either individual unit 10, 28, 48, 68, separately or all of the handheld glucose-insulin data management units (28, 48, 68) usable together in a disease management system. Further, the analyte measurement and management unit or DMU 10 is intended to include a glucose meter, a meter, an analyte measurement device, an insulin delivery device or a combination of an analyte testing and drug delivery device. In an embodiment, analyte measurement and management unit 10 may be connected to personal computer 26 with a cable. In an alternative, the DMU may be connected to the computer 26 or server 70 via a suitable wireless technology such as, for example, GSM, CDMA, BlueTooth, WiFi and the like.

Glucose meter or DMU 10 can include a housing 11, user interface buttons (16, 18, and 20), a display 14, a strip port connector 22, and a data port 13, as illustrated in Figure 1A. User interface buttons (16, 18, and 20) can be configured to allow the entry of data, navigation of menus, and execution of commands. Data can include values representative of analyte concentration, and/or information, which are related to the everyday lifestyle of an individual. Information, which is related to the everyday lifestyle, can include food intake, medication use, occurrence of health check-ups, and general health condition and exercise levels of an individual. Specifically, user interface buttons (16, 18, and 20) include a first user interface button 16, a second user interface button 18, and a third user interface button 20. User interface buttons (16, 18, and 20) include a first marking 17, a second marking 19, and a third marking 21, respectively, which allow a user to navigate through the user interface.

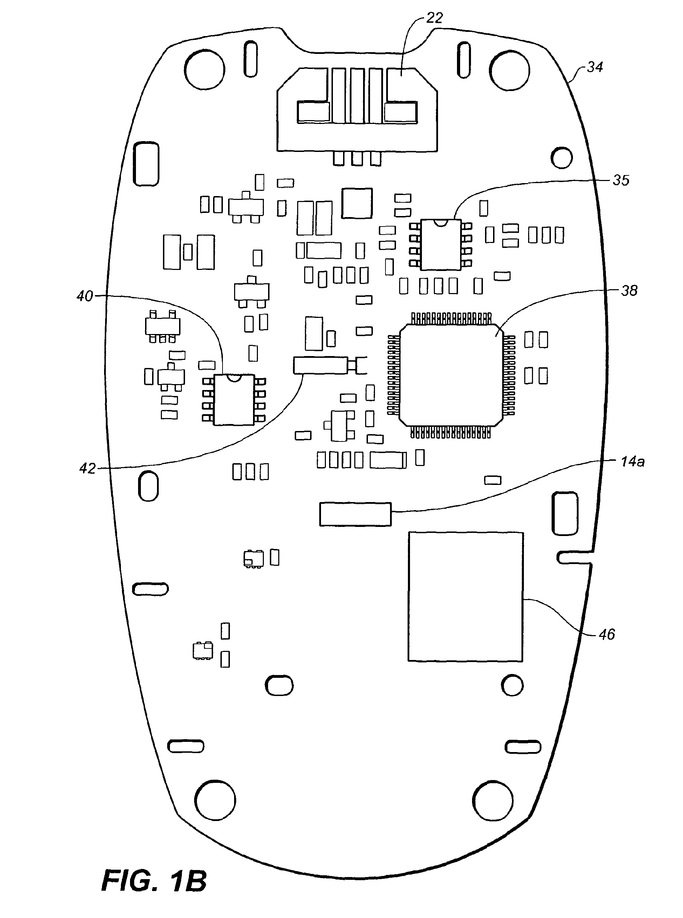

The electronic components of meter 10 can be disposed on a circuit board 34 that is within housing 11. Figure 1B illustrates (in simplified schematic form) the electronic components disposed on a top surface (not shown) of circuit board 34, respectively. On the top surface, the electronic components include a strip port connector 22, an operational amplifier circuit 35, a microcontroller 38, a display connector 14a, a non-volatile memory 40, a clock 42, and a first wireless module 46. Microcontroller 38 can be electrically connected to strip port connector 22, operational amplifier circuit 35, first wireless module 46, display 14, non-volatile memory 40, clock 42, and user interface buttons (16, 18, and 20).

Operational amplifier circuit 35 can include two or more operational amplifiers configured to provide a portion of the potentiostat function and the current measurement function. The potentiostat function can refer to the application of a test voltage between at least two electrodes of a test strip. The current function can refer to the measurement of a test current resulting from the applied test voltage. The current measurement may be performed with a current-to-voltage converter. Microcontroller 38 can be in the form of a mixed signal microprocessor (MSP) such as, for example, the Texas Instrument MSP 430. The MSP 430 can be configured to also perform a portion of the potentiostat function and the current measurement function. In addition, the MSP 430 can also include volatile and non-volatile memory. In another embodiment, many of the electronic components can be integrated with the microcontroller in the form of an application specific integrated circuit (ASIC).

Strip port connector 22 can be configured to form an electrical connection to the test strip. Display connector 14a can be configured to attach to display 14. Display 14 can be in the form of a liquid crystal display for reporting measured glucose levels, and for facilitating entry of lifestyle related information. Display 14 can include a backlight. A data port can be provided to accept a suitable connector attached to a connecting lead, thereby allowing glucose meter 10 to be linked to an external device such as a personal computer. The data port can be any port that allows for transmission of data such as, for example, a serial, USB, or a parallel port. Clock 42 can be configured to keep current time related to the geographic region in which the user is located and also to measure time. The DMU can be configured to be electrically connected to a power supply such as, for example, a battery.

For example, test strip 24 can be in the form of an electrochemical glucose test strip. Test strip 24 can include one or more working electrodes and a counter electrode. Test strip 24 can also include a plurality of electrical contact pads, where each electrode can be in electrical communication with at least one electrical contact pad. Strip port connector 22 can be configured to electrically interface to the electrical contact pads and form electrical communication with the electrodes. Test strip 24 can include a reagent layer that is disposed over at least one electrode. The reagent layer can include an enzyme and a mediator. Exemplary enzymes suitable for use in the reagent layer include glucose oxidase, glucose dehydrogenase (with pyrroloquinoline quinone co-factor, "PQQ"), and glucose dehydrogenase (with flavin adenine dinucleotide co-factor, "FAD"). An exemplary mediator suitable for use in the reagent layer includes ferricyanide, which in this case is in the oxidized form. The reagent layer can be configured to physically transform glucose into an enzymatic by-product and in the process generate an amount of reduced mediator (e.g., ferrocyanide) that is proportional to the glucose concentration. The working electrode can then measure a concentration of the reduced mediator in the form of a current. In turn, glucose meter 10 can convert the current magnitude into a glucose concentration. Details of the preferred test strip are provided in U.S. Patent Nos. 6179979; 6193873; 6284125; 6413410; 6475372; 6716577; 6749887; 6863801; 6890421; 7045046; 7291256; 7498132.

Referring back to Figure 1A, insulin pen 28 can include a housing, preferably elongated and of sufficient size to be handled by a human hand comfortably. The device 28 can be provided with an electronic module 30 to record dosage amounts delivered by the user. The device 28 may include a second wireless module 32 disposed in the housing that, automatically without prompting from a user, transmits a signal to first wireless module 46 of the DMU 10. The wireless signal can include, in an exemplary embodiment, data to (a) type of therapeutic agent delivered; (b) amount of therapeutic agent delivered to the user; or (c) time and date of therapeutic agent delivery.

In one embodiment, a therapeutic delivery device can be in the form of a "user-activated" therapeutic delivery device, which requires a manual interaction between the device and a user (for example, by a user pushing a button on the device) to initiate a single therapeutic agent delivery event and that in the absence of such manual interaction delivers no therapeutic agent to the user. A non-limiting example of such a user-activated therapeutic agent delivery device is described in co-pending U.S. Non-Provisional Application No. 12/407173 (tentatively identified by Attorney Docket No. LFS-5180USNP); 12/417875 (tentatively identified by Attorney Docket No. LFS-5183USNP); and 12/540217 (tentatively identified by Attorney Docket No. DDI-5176USNP). Another non-limiting example of such a user-activated therapeutic agent delivery device is an insulin pen 28. Insulin pens can be loaded with a vial or cartridge of insulin, and can be attached to a disposable needle. Portions of the insulin pen can be reusable, or the insulin pen can be completely disposable. Insulin pens are commercially available from companies such as Novo Nordisk, Aventis, and Eli Lilly, and can be used with a variety of insulin, such as Novolog, Humalog, Levemir, and Lantus.

Referring to Figure 1A, a therapeutic dosing device can also be a pump 48 that includes a housing 50, a backlight button 52, an up button 54, a cartridge cap 56, a bolus button 58, a down button 60, a battery cap 62, an OK button 64, and a display 66. Pump 48 can be configured to dispense medication such as, for example, insulin for regulating glucose levels.

Each individual measurement made by a patient may be stored in the memory of the DMU 10, and may be flagged with the corresponding date and time, and with additional flagging such as pre-meal and post-meal as it may be useful to look at how blood glucose varies around a given meal time, on average, over several consecutive days. Certain time periods may be programmed into the software of the meter to ensure that each measurement falls within a particular slot, for example before breakfast, after breakfast, before lunch, after lunch, before dinner or after dinner. It is an aspect of the present invention to provide messaging to the patient regarding the management of their condition, the specific messages depending upon the patient's measured data. However, according to the preferred embodiments, delivery of such messages to the patient may be dependent on there being a sufficient number of measured results stored within the memory of the device e.g. glucose monitoring meter, to be able to provide messaging with a certain level of confidence. In an example embodiment the required level of confidence e.g. 95% or 99% may be achieved by using a fixed significance level as well as a fixed level of statistical power, as will be described in more detail in relation to the following figures.

If a threshold difference in pre- and post-prandial glucose concentrations $D$ is set for differential messaging, then the issue of statistical significance and power arises. False positives (no difference in averages pre- and post-prandial but message says there is) and false negatives (there is a difference between pre- and post-prandial averages, but message says there isn't) can be misleading and potentially dangerous to the patient. The risk of such events therefore needs to be controlled.

The power of a statistical test is the probability that the test will reject a false null hypothesis (i.e. a false negative). As the power increases, then the probability of incorrectly rejecting a null hypothesis decreases. Performing a power analysis prior to data collection is typically used to determine an appropriate sample size to achieve adequate power. The power of a statistical test is the probability that the test will find a significant difference between the sample populations. The statistical power is dependent upon the significance level of the test and the sample size, therefore one way to increase the power of a test (i.e. increase the chance of correctly rejecting a null hypothesis when it is false) is to increase or weaken the significance level. However, weakening the significance level can increase the risk of obtaining a statistically significant result when the null hypothesis is in fact true i.e. obtaining a false positive result.

Further, as a user gains control of their diabetes with time, the magnitude of the pre/post-prandial average shift in glucose that they wish to detect should diminish. Given that the significance level ($\alpha$) controls the number of false positives, and the statistical power (1-$\beta$) controls the number of false negatives, then the statistical power of the test i.e. the probability of the user obtaining a false negative, would vary if a fixed sample size were to be used. Providing a patient with a false negative could be potentially dangerous in that they could take action such as injecting themselves with insulin, which may be an inappropriate action to take if the result or message provided to them was incorrect. It is the intent of applicant therefore to fix the significance level and statistical power of a statistical test analyzing the measured data, and allowing the number of measurements including the sample size, used to determine the result provided to the patient, to vary.

In general terms, the DMU 10 monitors pre- and post-prandial glucose results, for each mealtime, for a period until the standard deviation (SD) of the difference $D$ between each pair of pre- and post-prandial measured results can be estimated with reasonable confidence. The 95% Confidence Interval for a SD is provided by:

$$s\sqrt{\frac{n-1}{\chi^2_{n-1}(0.975)}} < \sigma < s\sqrt{\frac{n-1}{\chi^2_{n-1}(0.025)}}$$

where

$\sigma$ is the true SD,
n is the sample size,
$s$ is the estimated (measured value) SD, and
$\chi$ values refer to tabulated values of statistical distributions.

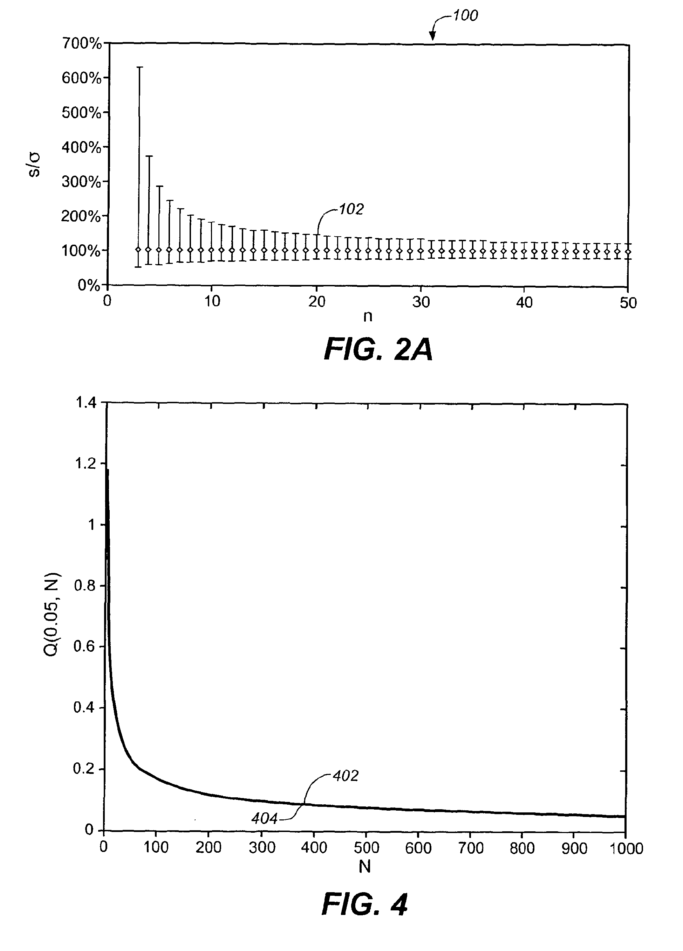

While there is no fixed rule for the sample size "n", from Figure 2A, it can be seen that for a sample size of n=20 (proximate the area noted by arrow 102) or more should provide convergence in the intervals. However, normal use of such statistical calculations would result in associated type 1 and type 2 errors i.e. providing the patient with a false negative results or a false positive result.

Applicants therefore propose, in this disclosure, to implement a method of testing pre-/post prandial glucose shifts, on a meal-by-meal basis, which will always maintain a fixed statistical power and significance.

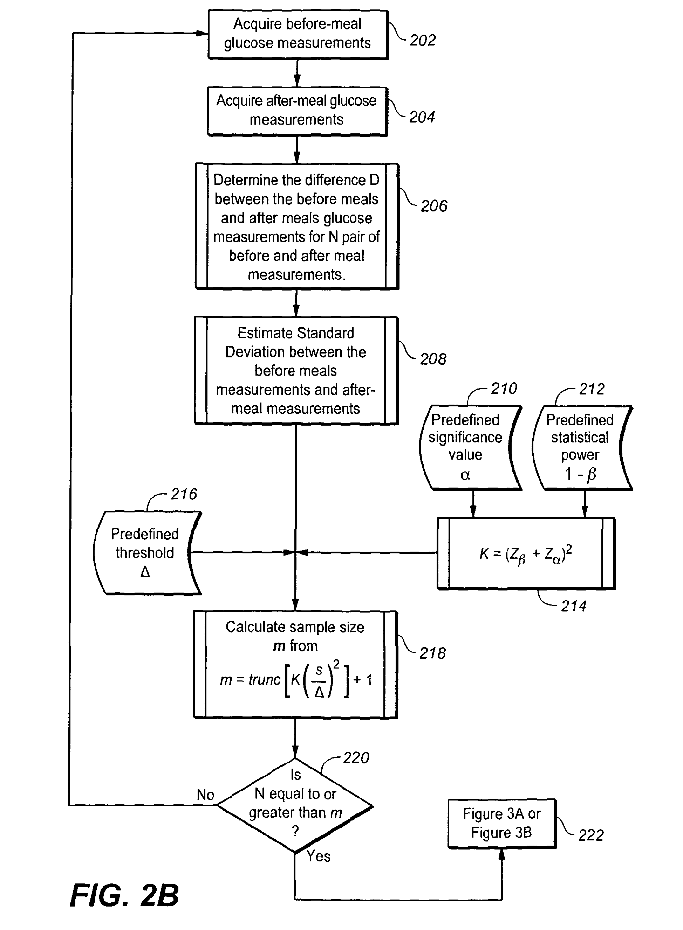

From Figure 2B, measured results of pre-prandial glucose concentration (e.g. a predefined period before meals, such as 2 hours for example) are obtained by the patient, using a DMU 10, and subsequently stored in the memory of the monitoring device at step 202. Next, a corresponding post-prandial glucose measurement (e.g. a predefined period after meals, such as 2 hours for example) is acquired and also stored, step 204. Then the difference **D** between the pre- and post-prandial measurements is calculated and stored, step 206. Difference value **D** gives an indication of the shift in glucose concentration experienced by the patient over a certain meal time e.g. lunch. It is typical for a diabetic patient's glucose concentration to increase after consuming food, as their body is unable to make the insulin required to maintain more stable blood sugar levels. These periods of high glucose concentration, or 'spikes' are periods of hyperglycemia which may be potentially damaging to the longer term health of the patient, due to an increased risk of diabetes-related conditions such as microvascular disease that can lead to retinopathy (eye disease), neuropathy (nerve damage) and nephropathy (kidney disease), circulatory problems, heart attacks and strokes for example.

Additionally, the number N of pairs of pre- and post-prandial measurement results comprising the sample size may be statistically analysed to determine whether the sample contains a sufficient number of data points to allow the standard deviation (SD) of the differences **D** to be estimated with the required level of statistical confidence e.g., 95%, step 208.

All individual measured results as well as the calculated difference **D** there-between, and standard deviation 's', may be stored within the memory of the device DMU 10. In one embodiment, one threshold difference value $\Delta$ may be stored and used for comparison of measured data according to any mealtime. In another embodiment, more than one threshold difference value $\Delta$ may be stored: thereby each individual mealtime may have a specific corresponding threshold difference value$\Delta$, step 216.

Threshold value $\Delta$ at step 216 may be initially set to a default value within the device e.g., glucose meter or mobile phone, during manufacture, and may be user or HCP configurable to more closely meet the individual management regimes of individual patients. The threshold difference value may also be configurable to more closely manage the patient's blood glucose concentration as it is brought under control.

A significance level $\alpha$ for the 1-sided t-test to detect a difference of at least the preset threshold value $\Delta$ is provided, step 210, as is a level of statistical power 1 - $\beta$, step 212. Significance level $\alpha$ and statistical power (1 - $\beta$) may, in one example embodiment of the present invention, be predefined at the time of manufacture, and may not be configurable by the user or HCP for example. In other embodiments, $\alpha$ and (1 - $\beta$) may be configurable.

In order to detect a difference for each N pair of pre and post prandial measurements above at least the predefined threshold value $\Delta$, with a predefined significance level ($\alpha$) and statistical power (1 - $\beta$), then the effect of $\alpha$ and $\beta$ on sample size 'm' can be implemented in the form of a pre-calculated constant 'K', at step 214:

$$K = \left(z_\beta + z_\alpha\right)^2 \qquad \text{Eq. 1}$$

where the respective z-values correspond to values of a normal variate with the appropriate probabilities. Such statistical methods are well-known in the art, and will not be described in further detail herein.

EXAMPLE 1

In one illustrative embodiment, use of a specified power of 80% (a standard level typically used for adequacy) provides a value of $\beta$ = 1 - 80/100 = 0.2, which generates a value $z_\beta$ = 0.8416 from the appropriate standard statistical tables or functions. Furthermore, for a 100$\alpha$% 1-tailed test, with $\alpha$ = 5% (i.e. 5% significance), $z_{\alpha/2}$ =1.6449 obtained also from statistical tables. Therefore, following the equation for 'K' set out above, this example provides a constant value of,

$$K = \left(0.8416 + 1.6449\right)^2 = 2.4865^2 = 6.186 \quad \text{Eq. 2}$$

In order to detect the preset threshold value $\Delta$ with the specified power, at the specified significance level, the sample size 'm' for the statistical test may therefore be at least:

$$m = K\left(\frac{\sigma}{\Delta}\right)^2 \approx K\left(\frac{s}{\Delta}\right)^2 \qquad \text{Eq. 3}$$

Generally this is not an integer, so a more common usage is, step 218:

$$m = trunc\left[K\left(\frac{s}{\Delta}\right)^2\right]+1 \qquad \text{Eq. 4}$$

Continuing with the present example, given the constant 'K' just calculated in step 218, the minimum sample size 'm' required to detect a threshold value of $\Delta$ that is half the standard deviation (SD) may be calculated as:

$$m = trunc\left[6.186 \times 2^2\right]+1 = 24 +1 = 25 \qquad \text{Eq. 5}$$

Where a number of N pairs of pre and post prandial measurements for a particular meal event (e.g., lunch) are equal to or greater than the value m then a suitable statistical test (e.g., a 1-tailed test) can be used to ascertain whether the threshold value has been exceeded with an acceptable level of certainty, step 220.

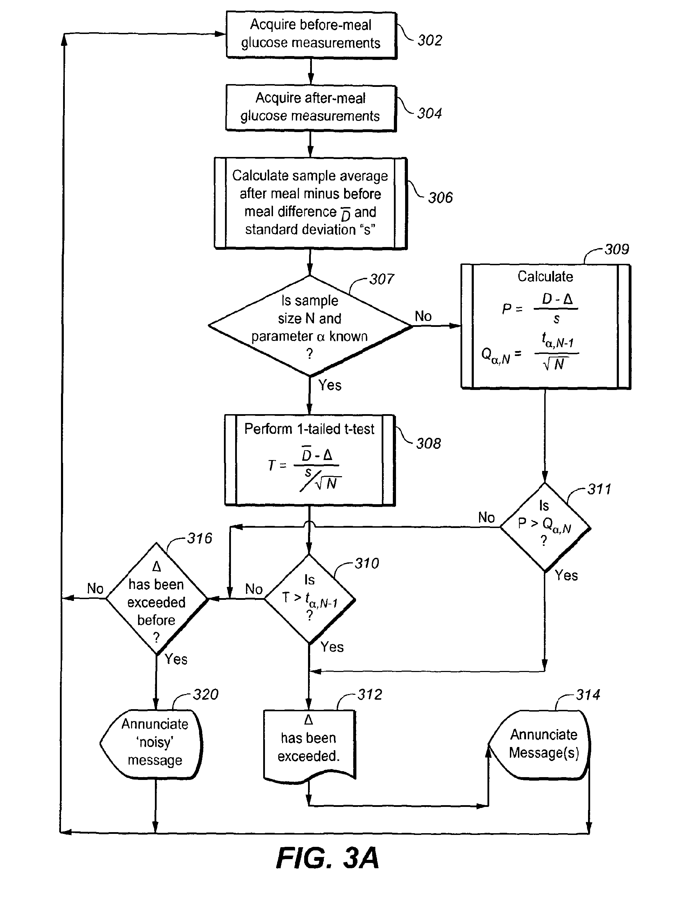

Figure 3A illustrates the steps involved in determining the sample size 'N' required in order to be able to detect a difference of at least a predefined threshold value $\Delta$, whilst also meeting the predefined significance level $\alpha$ and statistical power (1 - P) as described in relation to Figure 2B.

Firstly, paired pre- and post-prandial glucose concentration measurements for a particular meal, such as, for example, lunch, are acquired by the patient and stored within the memory of their meter, steps 302 and 304. Next, a mean difference value $\overline{D}$ of the beforemeal and after-meal measurements and the standard deviation's' are calculated in step 306.

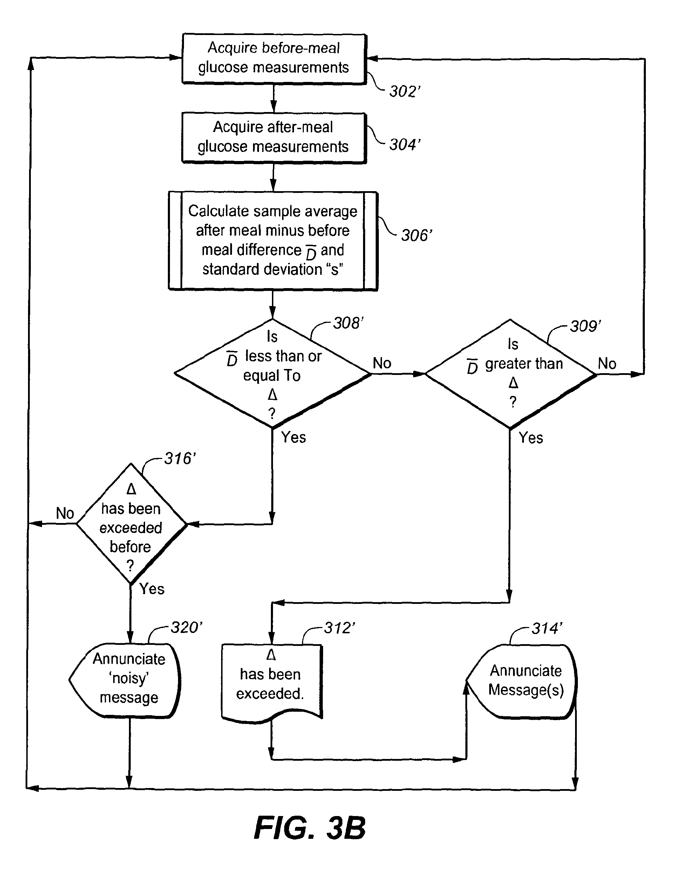

A statistical test typically used to detect whether a significant difference exists between two mean values may be a 1-tailed t-test. This is normally shown as a null hypothesis $H_0$, where in one example embodiment, the mean pre/post-prandial difference $\overline{D}$ is less than or equal to the threshold value $\Delta$ can be inferred in step 310 (or step 308' in Figure 3B), and an alternative hypothesis $H_1$, where the mean difference $\overline{D}$ is greater than the threshold value $\Delta$, step 312 (or step 309' in Figure 3B). In order to determine if the 1-tailed test can be utilized, a logical operator at step 307 determines if sample size N and significance level $\alpha$ are known. If true then the logic flows from step 307 to step 308.

Thereafter, a 1-tailed t-test using mean '$\overline{D}$', standard deviation 's' and threshold value $\Delta$ may then be carried out, at step 308. For example, the test quantity T may be calculated as:

$$T = \frac{\overline{D} - \Delta}{\left(s/\sqrt{N}\right)} \qquad \text{Eq. 6}$$

where $\overline{D}$ is the sample average post-prandial minus pre-prandial difference, calculated at step 306. The value of $T$ obtained is then compared against a critical value $t_{\alpha,N-1}$ derived from an appropriate statistical table for the t-test. Table 1 shows an example of an appropriate statistical table for the t-test.

Table 1

| N | $\alpha$ | | | | |
|---|---------|---------|---------|---------|----------|
| | 10.0% | 5.0% | 2.5% | 1.0% | 0.5% |
| 2 | 6.314 | 12.706 | 25.452 | 63.657 | 127.321 |
| 3 | 2.920 | 4.303 | 6.205 | 9.925 | 14.089 |
| 4 | 2.353 | 3.182 | 4.177 | 5.841 | 7.453 |

(continued)

| N | 10.0% | 5.0% | 2.5% | 1.0% | 0.5% |
|---|-------|------|------|------|------|
| | | | $\alpha$ | | |
| 5 | 2.132 | 2.776 | 3.495 | 4.604 | 5.598 |
| 6 | 2.015 | 2.571 | 3.163 | 4.032 | 4.773 |
| 7 | 1.943 | 2.447 | 2.969 | 3.707 | 4.317 |
| 8 | 1.895 | 2.365 | 2.841 | 3.499 | 4.029 |
| 9 | 1.860 | 2.306 | 2.752 | 3.355 | 3.833 |
| 10 | 1.833 | 2.262 | 2.685 | 3.250 | 3.690 |
| 11 | 1.812 | 2.228 | 2.634 | 3.169 | 3.581 |
| 12 | 1.796 | 2.201 | 2.593 | 3.106 | 3.497 |
| 13 | 1.782 | 2.179 | 2.560 | 3.055 | 3.428 |
| 14 | 1.771 | 2.160 | 2.533 | 3.012 | 3.372 |
| 15 | 1.761 | 2.145 | 2.510 | 2.977 | 3.326 |
| 16 | 1.753 | 2.131 | 2.490 | 2.947 | 3.286 |
| 17 | 1.746 | 2.120 | 2.473 | 2.921 | 3.252 |
| 18 | 1.740 | 2.110 | 2.458 | 2.898 | 3.222 |
| 19 | 1.734 | 2.101 | 2.445 | 2.878 | 3.197 |
| 20 | 1.729 | 2.093 | 2.433 | 2.861 | 3.174 |
| 21 | 1.725 | 2.086 | 2.423 | 2.845 | 3.153 |
| 22 | 1.721 | 2.080 | 2.414 | 2.831 | 3.135 |
| 23 | 1.717 | 2.074 | 2.405 | 2.819 | 3.119 |
| 24 | 1.714 | 2.069 | 2.398 | 2.807 | 3.104 |
| 25 | 1.711 | 2.064 | 2.391 | 2.797 | 3.091 |
| 26 | 1.708 | 2.060 | 2.385 | 2.787 | 3.078 |
| 27 | 1.706 | 2.056 | 2.379 | 2.779 | 3.067 |
| 28 | 1.703 | 2.052 | 2.373 | 2.771 | 3.057 |
| 29 | 1.701 | 2.048 | 2.368 | 2.763 | 3.047 |
| 30 | 1.699 | 2.045 | 2.364 | 2.756 | 3.038 |

Typically, if $T$ exceeds a critical value $t_{\alpha, N-1}$ of the $t$-distribution set by the significance level $\alpha$ and the sample size $N$, then the null hypothesis $H_0$ is rejected in favour of the alternative hypothesis that $\Delta$ has been exceeded. According to an example embodiment of the present invention, significance level $\alpha$ may be predefined, however, the sample number 'N' may not be predefined or fixed at manufacture for example, and is therefore not known, and so cannot be used in the calculation. As noted before, this calculation depends upon knowing both significance level $\alpha$ and sample size 'N' in advance in order to be able to look up the appropriate t-distribution tables.

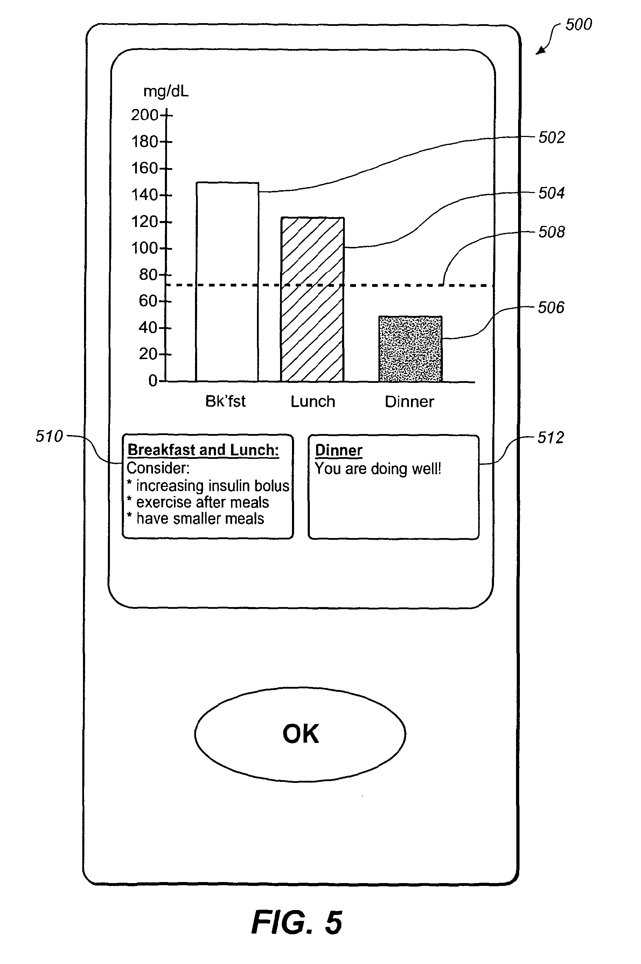

In one example embodiment, assuming $\alpha$ and N are both known, then the value or 'T' obtained is compared against the critical value $t_{\alpha, N-1}$ at $_{step}$ 310, and if T does exceed the critical value $t_{\alpha, N-1}$ then the null hypothesis $H_0$ is rejected in favour of an alternative hypothesis $H_1$, step 312 and an appropriate message may be displayed to the user at step 314. An example of messages displayed to the user in shown and discussed in relation to Figure 5. However, if T does not exceed the critical value $t_{\alpha, N-1}$ in step 310, then a query, at step 316, is made to determine if the value $\Delta$ has been exceeded before. If true at step 316 then the logic accepts the hypothesis H1, and appropriate messaging may be displayed to the user, at step 320. If not true at step 316, i.e. the value $\Delta$ has not been exceeded before, then the null hypothesis $H_0$ is accepted and the logic returns to 302.

Alternatively, however, the sample size 'N' may not be known or predefined. The number of measurement results comprising the sample size is variable to ensure that a difference of at least the threshold value $\Delta$ can be detected from the standard deviation of the differences between pre- and post-prandial glucose measurements, with the required confidence interval. In such case, the logic flow at step 307 returns a false which means that this leaves outstanding the problem of how to obtain the critical value $t_{\alpha,N-1}$ described above. There are many ways to achieve this, an example of which is given here, making use of a re-statement of the problem. Comparing $T$ with the critical value $t_{\alpha,N-1}$ is equivalent to comparing the quantity P, and the critical value $Q_{\alpha,N}$, which are determined as follows:

$$P = \frac{\overline{D} - \Delta}{s} \qquad \text{Eq. 7}$$

with the critical value,

$$Q_{\alpha,N} = \frac{t_{\alpha,N-1}}{\sqrt{N}} \qquad \text{Eq. 8}$$

In the case where $\alpha$, = 5%, at step 309, the estimated critical value $Q_{\alpha,N}$ is well approximated by the simple function shown here in Figure 4:

$$Q_{0.05,N} = 1.9182 \times N^{-0.5312} \qquad \text{Eq. 9,}$$

even for very large values of $N$, and the function is shown in Figure 4. In Figure 4, line 402 is generated using Equation 8 where several values of $Q_{0.05,N}$ are calculated as a function of $t_{0.05,N}$ and N. Based on the shape of line 402, applicant chose a function $Y = a \times N^{-b}$ as an empirical framework for approximating line 402. After applying a minimization software routine to the empirical function $Y = a \times N^{-b}$, Equation 10 with the associated constants for a and b were derived. Note that the use P and $Q_{0.05,N}$ does not require a t-table to be stored into memory or the calculation of the t-table, which is computationally and/or memory intensive process within a hand-held device. Thus, the use of P and $Q_{0.05,N}$ is computationally simpler than the use of T and $t_{0.05,N}$ and consequently reduces the amount of microprocessing power and memory.

The following shows an example of the computationally intensive calculations required for estimating a value of t. In Equation 10, $f_n(t)$ is the probability density function for the Student's t variate with n-1 degrees of freedom.

$$f_n(t) = \frac{\Gamma\left(\dfrac{N}{2}\right)}{\sqrt{(N-1)\pi}\,\Gamma\left(\dfrac{N-1}{2}\right)\left(1 + \dfrac{t^2}{N-1}\right)^{\frac{N}{2}}} \qquad \text{Eq. 10}$$

The term $\Gamma(z) = \displaystyle\int_0^\infty u^{z-1} e^{-u}\, du$ is the gamma function. X can be the desired critical value for a one sided significance test with significance level $100\alpha\%$ then X is the solution of the integral equation $F_n(X) = \displaystyle\int_{-\infty}^{x} f_n(t)\,dt = 1 - \alpha$ The function $F_n(t)$ is the cumulative distribution function of the t variate, and may be calculated using the relation $F_n(t) = 1 - I_{\frac{N-1}{N-1+t^2}}\left(\dfrac{N-1}{2}, \dfrac{1}{2}\right)$, where $I_x\,(a,\, b)$ is the incomplete beta function. For reference, the computer code to evaluate the incomplete beta function numerically may be viewed at <u>http://www.fizy-ka.umk.pl/nrbook/c6-4.pdf</u>, part of an on-line version of the book "Numerical Recipes in C" illustrating the complexity of the t calculation.

As noted earlier with reference to Figure 2B, the standard deviation (SD) is first calculated, and then the minimum sample size '*m*', and then waiting until m pairs of pre-and post-prandial readings have been taken, allow the power of the test to be controlled. Varying the sample size '*m*' in this way in order to maintain a fixed statistical power $(1 - \beta)$ enables the probability of a false negative being displayed to the patient to be minimized. Thereafter, with reference to Figure 3A, the method calculates the quantity P and derives an equivalent critical value $Q_{0.05,N}$ in step 309 (from Equation. 8-9).

The methods described herein can be used to control the statistical power regardless of how variable an individual's readings may be (how large the SD is). As a patient gains control of their condition with time, then the magnitude of the pre- /post-prandial average glucose shift to be detected will diminish, however the statistical power i.e. the control of false negatives can still be maintained by modifying the number of measurement results comprising the sample size '*m*'. It is likely that the size of sample size '*m*' may vary in relation to the relative size of the predefined threshold value $\Delta$ to be detected i.e. a smaller pre- / post-prandial average glucose shift may require a smaller sample size 'N' to detect the difference whilst still meeting the required confidence interval.

Figure 4 is a plot 400 showing a comparison of the true function for $Q_{\alpha,N}$ 402 compared against the simple approximation 404 generated from Equation 9

Figure 4 shows how closely the simple approximation 404 matches the true function for the critical value function 402 with a significance level $\alpha$ of 5%. Simple approximation 404 closely matches the curve obtained for the true function 402 over a wide range of sample sizes 'N', including very small sample sizes as well as very large sample sizes. For the purposes described herein, it is reasonable to assume that the simple approximation 404 is equal to the true function 402, and therefore the values of sample size 'N' can be calculated.

The final step is to make use of which hypothesis, outlined in the description related to Figure 3A, is being supported by the test, and then to choose appropriate messaging accordingly, as described in relation to Figure 5. Where the logical operation at 310 determines that T is greater than the critical value $t_{\alpha,N-1}$ then a conclusion is made at step 312 the threshold $\Delta$ has been exceeded (and the alternative hypothesis $H_1$ is selected) and a message such as 510 in Figure 5 is displayed. On the other hand, if T is less than or equal to the critical value $t_{\alpha,N-1}$ at step 310 then a determination is made at step 316 that the threshold $\Delta$ has not been exceeded (hence the null-hypothesis is selected).

In alternative embodiment where a simplified algorithm of Figure 3A is intended, the process flow of Figure 3B may be utilized for each meal events over a period of time. Firstly, paired pre- and post-prandial glucose concentration measurements for a particular meal, such as, for example, lunch, are acquired by the patient and stored within the memory of their meter, steps 302' and 304'. Next, a mean difference value $\overline{D}$ of the before-meal and after-meal measurements and the standard deviation's' are calculated in step 306'. This is normally shown as a null hypothesis $H_0$, step 308', where in one example embodiment, the mean pre/post-prandial difference $\overline{D}$ is less than or equal to the threshold value $\Delta$, and an alternative hypothesis $H_1$, step 309', where the mean difference $\overline{D}$ is greater than the threshold value $\Delta$, at step 312'. Where the logical operation at 308' determines that the mean pre/post-prandial difference $\overline{D}$ is less than or equal to the threshold value $\Delta$ then a query is made at step 316' as to whether the threshold $\Delta$ has been exceeded before. If the threshold has been exceed before at step 316' then the null-hypothesis is selected otherwise and an appropriate message is displayed at step 320'. Otherwise, if the threshold has not been exceeded before then the logic returns to step 302'. Where step 308' returns a no, a determination is made as to whether $\overline{D}$ is greater than the threshold value $\Delta$, at step 312'. If true, then a conclusion is made at step 312 the threshold $\Delta$ has been exceeded (and the alternative hypothesis $H_1$ is selected) and a message such as 510 in Figure 5 is displayed.

The flow diagram of Figure 3A or 3B outlines the main process steps that may take place within an embodiment of the software algorithm of the present invention. As described previously, applicants intend to enable the number of pre- / post-prandial measured results i.e. the sample size of the number of pre- / post-prandial differences held in the memory of the device, to vary thereby enabling the significance level and statistical power of the statistical test to be predetermined i.e. fixed during manufacture. Fixing the significance level and power of a statistical test ensures that the comparison between the standard deviation of the pre- / post-prandial differences and a predefined threshold value $\Delta$ can be detected with reasonable confidence i.e. within the required confidence interval. Controlling the statistical power may also minimize the number of false negative results displayed to the user which could cause them to take inappropriate action with regard to their diabetes management regime.

The Type 1 (false positive results) and Type 2 (false negative results) error rates associated with a test can be controlled within the DMU 10, by ensuring that a sufficiently large sample size is used to give both a stable estimate of the standard deviation, and to provide adequate power in a t-test to determine whether or not an acceptable threshold average difference $\Delta$ between pre- and post-prandial has been exceeded on a meal-by-meal basis.

## EXAMPLE 2

Pre and post-prandial blood glucose measurements (in units of milligrams per deciliter) were collected from a user over a twenty day period, as illustrated in Table 2. The columns "day," "before" and "after" in the main body of the table enumerate days, list pre- and post-prandial measurements, respectively.

Table 2

| K= | 6.186 | | | | | | | | | | |
|---|---|---|---|---|---|---|---|---|---|---|---|
| DELTA= | 7.5 | mg/dL | | | | | | | | | |
| | | | | | | | | | | | |
| | | | | | | | | | | | |
| | | | | | | | | | | | |
| | | | | | | | | | | | |
| day | before | after | D | s | m | N | ready | Dbar | P | Q | sig? |
| 1 | 80 | 105 | 25 | | | | | | | | FALSE |
| 2 | 84 | 98 | 14 | 7.78 | 7 | 2 | FALSE | | | | FALSE |
| 3 | 83 | 115 | 32 | 9.07 | 10 | 3 | FALSE | | | | FALSE |
| 4 | 83 | 103 | 20 | 7.63 | 7 | 4 | FALSE | | | | FALSE |
| 5 | 75 | 104 | 29 | 7.18 | 6 | 5 | FALSE | | | | FALSE |
| 6 | 76 | 114 | 38 | 8.59 | 9 | 6 | FALSE | | | | FALSE |
| 7 | 83 | 103 | 20 | 8.20 | 8 | 7 | FALSE | | | | FALSE |
| 8 | 93 | 102 | 9 | 9.56 | 11 | 8 | FALSE | | | | FALSE |
| 9 | 84 | 109 | 25 | 8.96 | 9 | 9 | FALSE | | | | FALSE |
| 10 | 83 | 98 | 15 | 8.87 | 9 | 10 | TRUE | 22.70 | 1.71 | 0.565 | TRUE |
| 11 | 83 | 104 | 21 | 8.43 | 8 | 11 | TRUE | 22.55 | 1.78 | 0.537 | TRUE |
| 12 | 79 | 112 | 33 | 8.59 | 9 | 12 | TRUE | 23.42 | 1.85 | 0.512 | TRUE |
| 13 | 92 | 95 | 3 | 9.98 | 11 | 13 | TRUE | 21.85 | 1.44 | 0.491 | TRUE |
| 14 | 80 | 103 | 23 | 9.60 | 11 | 14 | TRUE | 21.93 | 1.50 | 0.472 | TRUE |
| 15 | 82 | 99 | 17 | 9.33 | 10 | 15 | TRUE | 21.60 | 1.51 | 0.455 | TRUE |
| 16 | 76 | 102 | 26 | 9.08 | 10 | 16 | TRUE | 21.88 | 1.58 | 0.440 | TRUE |
| 17 | 77 | 97 | 20 | 8.81 | 9 | 17 | TRUE | 21.76 | 1.62 | 0.426 | TRUE |
| 18 | 90 | 113 | 23 | 8.55 | 9 | 18 | TRUE | 21.83 | 1.68 | 0.413 | TRUE |
| 19 | 88 | 114 | 26 | 8.36 | 8 | 19 | TRUE | 22.05 | 1.74 | 0.401 | TRUE |
| 20 | 87 | 96 | 9 | 8.65 | 9 | 20 | TRUE | 21.40 | 1.61 | 0.391 | TRUE |

The following will describe the calculations for determining whether the mean difference between pre and post-prandial glucose measurements is significant at a given day. If there is a significant mean difference between pre and post-prandial glucose measurements, then an indication can be displayed on a glucose meter screen such as a display device 500, as illustrated in Figure 5. If the data in Table 2 corresponds to having only one type of meal type (e.g., breakfast, lunch, or dinner), then the output of Table 2 can be used for generating one of the graphical bars (502, 504, or 506) of Figure 5.

As an initial part of the calculation, the value K is set to 6.186 and DELTA ($\Delta$) is set to 7.5 mg/dL. Note that the value K denoted in the top row of Table 2 is calculated with Eq. 2 for a t-test with 5% significance ($\alpha$), and 80% power ($\beta$). The second row of Table 2 gives a value of DELTA ($\Delta$), which is a minimum level of difference between pre- and post-prandial mean levels for determining a significant difference. The column "D" includes the differences between pre- and post-prandial measurements (i.e., "after" - "before") in units of milligrams per deciliter for each day (as described in step 206). The column "s" includes the standard deviation (SD) of all D values up to and including the day of the particular row. Note that the first row has no SD value because a minimum of 2 values are required to calculate SD. The column "m" includes the calculated sample size m required for the significance test on that day using Equation 4 with the specified K, s and $\Delta$. The column "N" includes the actual sample size N for that day. Note that in Table 2, N correlates with the number of days. However, in other situations more or less than one pre- and post-prandial measurement pair can be collected per day.

Once the value of N exceeds the computed value of m (which first happens on day 10 of Table 2), the t-test equivalent can be performed with the pre-defined significance and power. The "ready" column includes a "FALSE" where N is less than m, and a "TRUE" where N is greater than or equal to m.

After establishing that there are a sufficient number of pre- and post-prandial measurements (i.e., a day in which N is greater than or equal to m (i.e., N≥m)), the following columns of Table 2 will show whether the mean difference of pre- and post-prandial measurements is significant. The column "Dbar" includes the computed value of the average differences D up to and including the day of that particular row. The columns "P" and "Q" show the computed values of these quantities, as defined in Equations 7 and 9, respectively. The column "sig?" queries whether P > Q. If so, the test has revealed a statistically significant difference in mean pre- and post-prandial glucose (with the pre-defined significance and power). In this example, the mean differences are significant from day 10 onwards, so "sig?" is TRUE for days 10 through 20. A warning indication that the mean differences are significant can be shown on a display device 500, which is described below (see Figure 5).

Figure 5 shows an example embodiment of a message displayed to a patient with a suitable portable diabetes management display device 500. Display device 500 includes a graphical indication of the pre- / post-prandial differences measured and stored in the memory of a microprocessor a measure of the patient's glucose level shifts over each mealtime.

In the example embodiment shown in Figure 5, the results may be grouped according to specific meal times, e.g. pre- and post-breakfast 502, pre- and post-lunch 504, and pre-and post-dinner 506. Dashed line 508 may be used to indicate a target or threshold pre-/ post-prandial difference value as shown in Figure 5, where one target difference value of approximately 75mg/dL is given. More than one target difference value may be used according to different meal times for example. Alternatively, the use of different colours may be used to indicate more easily and quickly to the user (and/or HCP) whether the measured results are close to the threshold target value such as that defined by their HCP. In another embodiment, the use of different colours may be used to indicate more easily and quickly to the user (and/or HCP) whether the difference value is statistically significant based on Equations 7 and 9. For example a green colour may be used to show that the results are within the threshold value, whilst a red colour, for example, may be used to indicate results that differ significantly from the threshold value. It would be very easy for a patient and/or HCP to view messages of this type and gain an awareness of the patient's level of control of their diabetes. The use of colour in the graphical output displayed to the user is one, quick and easily understood way of comparing the measured results against a defined threshold value and/or indicating statistical significance in a computationally simple manner. Alternative modes of displaying the information would be possible and are intended to be included herein.

Figure 5 shows example data representing a patient's glucose difference measurements around breakfast 502, lunch 504 and dinner 506. The difference measurements around breakfast and lunch are shown to be highly variable in this example, and may in one embodiment be coloured red to quickly and easily identify to the patient and/or HCP that this may be an area requiring some attention. The example data shown to represent measured pre- / post-prandial glucose differences around dinner time 506 are well below the target value 508, and may therefore be coloured green for example to indicate that this particular component of the patient's measurement regime is well controlled.

Furthermore, display device 500 may include recommendations 510, 512 to the patient including but not restricted to medication, exercise and/or carbohydrate intake for example. In Figure 5, recommendation 510 refers to measured data around breakfast 502 and lunch 504, therefore specific suggestions may be made to bring the patient's glucose shifts closer to the defined target value 508 i.e. bring the patient into better control. Recommendation 512 may, in this example embodiment congratulate the patient for doing well.

As noted earlier, the microprocessor can be programmed to generally carry out the steps of various processes described herein. The microprocessor can be part of a particular device, such as, for example, a glucose meter, an insulin pen, an insulin pump, a server, a mobile phone, personal computer, or mobile hand held device. Furthermore, the various methods described herein can be used to generate software codes using off-the-shelf software development tools such as, for example, C, C+, C++, C-Sharp, Visual Studio 6.0, Windows 2000 Server, and SQL Server 2000. The methods, however, may be transformed into other software languages depending on the requirements and the availability of new software languages for coding the methods. Additionally, the various methods described, once transformed into suitable software codes, may be embodied in any computer-readable storage medium that, when executed by a suitable microprocessor or computer, are operable to carry out the steps described in these methods along with any other necessary steps.

An advantage of the present invention includes reducing the possibility of a patient being provided with a false negative result i.e. there is a significant difference between a patient's pre- and post-prandial glucose concentration averages, but the message provided to the patient says there isn't. Such a result could be misleading and potentially dangerous to the patient, should they take action guided by the result provided. The present invention therefore minimizes the risk of such events occurring by controlling the significance value $\alpha$ and the statistical power (1 - $\beta$), whilst allowing the number of pre- / post-prandial glucose measurements considered by the statistical calculation (i.e. the sample size) to vary in order to detect a significant difference from a predefined threshold value $\Delta$ within the required confidence interval.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above.

**Claims**

1. A method of alerting a user with a diabetes management device that the user's blood glucose data around a meal event has exceeded a predefined threshold value ($\Delta$), the method comprising:

   collecting, with the diabetes management device, a number of pre and post-prandial pairs (N) of glucose concentration measurements about a particular meal event;
   calculating, with a microprocessor of the diabetes management device, a plurality of differential values (D) based on a difference between each pair of the collected plurality of pre and post-prandial pairs of glucose concentrations about the particular meal event;
   determining a fixed significance level ($\alpha$) value and a fixed statistical power (1 - $\beta$) value;
   determining, with the microprocessor, wherein the number of plurality of pre and post-prandial pairs (N) is equal to or greater than a calculated sample size (m);
   ascertaining with at least one statistical test as to whether the threshold value ($\Delta$) has been exceeded with an acceptable level of certainty; and
   upon the ascertaining that the threshold value ($\Delta$) has been exceeded with an acceptable level of certainty, outputting to the user that for the particular meal, the plurality of differential values D for the number of pairs of glucose measurements has exceeded the threshold value ($\Delta$), wherein the effect of $\alpha$ and $\beta$ on sample size (m) is implemented in the form of a constant *K* defined as:

$$K = \left(z_\beta + z_\alpha\right)^2$$

   wherein z-values correspond to values of a Normal variate from statistical tables depending on the predefined significance level ($\alpha$) and the statistical power (1 - $\beta$),
   wherein the sample size m is calculated with an equation of the form:

$$m = trunc\left[K\left(\frac{s}{\Delta}\right)^2\right] + 1$$

   wherein *s* is a standard-deviation of pre and post prandial measurements and $\Delta$ is the predefined threshold value ($\Delta$).

2. The method of claim 1, in which the ascertaining comprises application of the statistical test of the form:

$$T = \frac{\overline{D} - \Delta}{\left(\frac{s}{\sqrt{N}}\right)}$$

   where

$\overline{D}$ is the mean difference value of the pre and post-prandial pairs of glucose concentrations;

s is the standard-deviation of pre and post prandial measurements; and

$\Delta$ is the predefined threshold value.

N is the number of plurality of pre and post-prandial pairs..

3. The method of claim 1, in which the ascertaining comprises calculating a quantity P and a critical value Q of the following respective forms:

$$P = \frac{\overline{D} - \Delta}{s}$$

and

$$Q_{\alpha,N} = \frac{t_{\alpha,N-1}}{\sqrt{N}}$$

where

$\overline{D}$ is the mean difference value of the pre and post-prandial pairs of glucose concentrations; $s$ is the standard-deviation of pre and post prandial
measurements; and

$\Delta$ is the predefined threshold value.

N is the number of plurality of pre and post-prandial pairs.; and

$t_{\alpha,N-1}$ is a critical value from a statistical table based on a significance level and a degree of freedom.

4. The method of claim 1, in which the ascertaining comprises calculating a quantity P and a critical value Q of the following respective forms:

$$P = \frac{\overline{D} - \Delta}{s}$$

$$Q_{0.05,N} = 1.9182 \times N^{-0.5312}$$

where

$\overline{D}$ is the mean difference value of the pre and post-prandial pairs of glucose concentrations;

s is the standard-deviation of pre and post prandial measurements; and

$\Delta$ is the predefined threshold value; and

N is the number of plurality of pre and post-prandial pairs..

5. The method according to any of the preceding claims, further comprising: performing a plurality of glucose concentration measurements to physically transform glucose in a fluid sample into an enzymatic by-product and generate an amount of reduced mediator (e.g., ferrocyanide) proportional to the glucose concentration of the fluid sample.

**Patentansprüche**

1. Verfahren zum Warnen eines Anwenders mit einer Diabetesmanagementeinheit, dass die Blutglucosedaten des Anwenders um ein Mahlzeitereignis einen vordefinierten Schwellenwert ($\Delta$) überschritten haben, wobei das Verfahren umfasst:

mit der Diabetesmanagementeinheit sammeln mehrerer prä- und postprandialer Paare (N) von Glucosekonzentration-Messungen zu einem bestimmten Mahlzeitereignis;

mit einem Mikroprozessor der Diabetesmanagementeinheit berechnen einer Vielzahl von Differenzwerten (D) auf der Grundlage des Unterschieds zwischen jedem Paar der gesammelten Vielzahl von prä- und postprandialen Paaren von Glucosekonzentrationen zu dem bestimmten Mahlzeitereignis;

Festlegen eines festen Werts des Signifikanzniveaus ($\alpha$) und eines festen Werts der statistischen Teststärke $(1 - \beta)$;

mit dem Mikroprozessor bestimmen, ob die Anzahl der Vielzahl von prä- und postprandialen Paaren (N) gleich wie oder größer als eine berechnete Probengröße (m) ist;

mit wenigstens einem statistischen Test sicherstellen, ob der Schwellenwert ($\Delta$) mit einem annehmbaren Sicherheitsniveau überschritten worden ist; und

bei Sicherstellen, dass der Schwellenwert ($\Delta$) mit einem annehmbaren Sicherheitsniveau überschritten worden ist, Ausgeben an den Anwender, dass für die bestimmte Mahlzeit die Vielzahl von Differenzwerten D für die mehreren Paare von Glucosemessungen den Schwellenwert ($\Delta$) überschritten hat,

wobei die Wirkung von $\alpha$ und $\beta$ auf die Probengröße (m) in der Form einer Konstanten K implementiert ist, die definiert ist als

$$K = \left( z_\beta + z_\alpha \right)^2$$

wobei z-Werte Werten einer Normal-Zufallsvariable aus statistischen Tabellen entspricht, die von dem vordefinierten Signifikanzniveau ($\alpha$) und der Teststärke $(1 - \beta)$ abhängt,

wobei die Probengröße m mit einer Gleichung der Form:

$$m = trunc \left[ K \left( \frac{s}{\Delta} \right)^2 \right] + 1$$

berechnet wird, wobei s eine Standardabweichung von prä- und postprandialen Messungen ist und $\Delta$ der vordefinierte Schwellenwert ($\Delta$) ist.

**2.** Verfahren gemäß Anspruch 1, wobei das Sicherstellen die Anwendung des statistischen Tests der Form:

$$T = \frac{\overline{D} - \Delta}{\left( \frac{s}{\sqrt{N}} \right)}$$

umfasst,
wobei

$\overline{D}$ der mittlere Differenzwert der prä-und postprandialen Paare von Glucosekonzentrationen ist;
s die Standardabweichung der prä- und postprandialen Messungen ist; und
$\Delta$ der vordefinierte Schwellenwert ist;
N die Anzahl der Vielzahl von prä- und postprandialen Paaren ist.

**3.** Verfahren gemäß Anspruch 1, wobei das Sicherstellen Berechnen einer Größe P und eines kritischen Werts Q der folgenden entsprechenden Formen umfasst:

$$P = \frac{\overline{D} - \Delta}{s}$$

und

$$Q_{\alpha,N} = \frac{t_{\alpha,N-1}}{\sqrt{N}}$$

wobei

$\overline{D}$ der mittlere Differenzwert der prä-und postprandialen Paare von Glucosekonzentrationen ist; s die Standardabweichung der prä- und postprandialen Messungen ist; und

$\Delta$ der vordefinierte Schwellenwert ist;

N die Anzahl der Vielzahl von prä- und postprandialen Paaren ist; und

$t_{\alpha,N-1}$ ein kritischer Wert aus einer statistischen Tabelle auf der Grundlage eines Signifikanzniveaus und eines Freiheitsgrads ist.

4. Verfahren gemäß Anspruch 1, wobei das Sicherstellen Berechnen einer Größe P und eines kritischen Werts Q der folgenden entsprechenden Formen umfasst:

$$P = \frac{\overline{D} - \Delta}{s}$$

$$Q_{0,05,N} = 1,9182 \times N^{-0,5312}$$

wobei

$\overline{D}$ der mittlere Differenzwert der prä-und postprandialen Paare von Glucosekonzentrationen ist;

s die Standardabweichung der prä- und postprandialen Messungen ist; und

$\Delta$ der vordefinierte Schwellenwert ist;

N die Anzahl der Vielzahl von prä- und postprandialen Paaren ist.

5. Verfahren gemäß einem der vorstehenden Ansprüche, ferner umfassend:

Durchführen einer Vielzahl von Messungen der Glucosekonzentration zum physischen Umwandeln von Glucose in einer Fluidprobe zu einem enzymatischen Nebenprodukt und Erzeugen einer Menge an reduziertem Mediator (beispielsweise Ferrocyanid) proportional zu der Glucosekonzentration in der Fluidprobe.

## Revendications

1. Procédé pour avertir un utilisateur muni d'un dispositif de gestion du diabète que les données de glycémie de l'utilisateur relativement à un repas ont dépassé une valeur de seuil prédéfinie ($\Delta$), le procédé comprenant les étapes suivantes :

collecter, avec le dispositif de gestion du diabète, un certain nombre de paires (N) de mesures de concentration de glucose pré- et postprandiales relativement à un repas particulier ;

calculer, avec un microprocesseur du dispositif de gestion du diabète, une pluralité de valeurs de différences (D) basées sur une différence entre chaque paire de la pluralité collectée de paires pré- et postprandiales de la glycémie relativement au repas particulier ;

déterminer une valeur de niveau de signification fixée ($\alpha$) et une valeur de puissance statistique fixée (1-$\beta$) ;

déterminer, avec le microprocesseur, dans le cas où le nombre de la pluralité de paires (N) pré- et postprandiales est égal ou supérieur à une taille d'échantillon calculé (m) ;

vérifier avec au moins un test statistique si la valeur de seuil ($\Delta$) a été dépassée avec un niveau acceptable de certitude ; et

lorsqu'il a été constaté que la valeur de seuil ($\Delta$) a été dépassée avec un niveau acceptable de certitude, délivrer à l'utilisateur que, pour le repas particulier, la pluralité de valeurs de différences D pour le nombre de paires de mesures de la glycémie ont dépassé la valeur de seuil ($\Delta$),

où l'effet de $\alpha$ et $\beta$ sur la taille de l'échantillon (m) est implémenté sous la forme d'une constante K définie par :

$$K = (z_\beta + z_\alpha)^2$$

où les valeurs de z correspondent à des valeurs d'une variante normale de tables statistiques en fonction du niveau de signification ($\alpha$) et de la puissance statistique (1-$\beta$) prédéfinis,
où la taille de l'échantillon m est calculée avec une équation de la forme suivante :

$$m = \text{valeur tronquée de } [K(s/\Delta)^2] + 1$$

où s est un écart-type de mesures pré- et postprandiales et $\Delta$ est la valeur de seuil ($\Delta$) prédéfinie.

2. Procédé selon la revendication 1, dans lequel la vérification comprend l'application du test statistique de la forme suivante :

$$T = \frac{\overline{D} - \Delta}{\left(\frac{s}{\sqrt{N}}\right)}$$

où $\overline{D}$ **est la valeur de différence moyenne** des paires pré- et postprandiales des glycémies ;
s est l'écart-type des mesures pré- et postprandiales ; et
$\Delta$ est la valeur de seuil prédéfinie,
N est le nombre de la pluralité de paires pré- et postprandiales.

3. Procédé selon la revendication 1, dans lequel la vérification comprend de calculer une quantité P et une valeur critique Q des formes respectives suivantes :

$$P = \frac{\overline{D} - \Delta}{s}$$

et

$$Q_{\alpha,N} = \frac{t_{\alpha,N-1}}{\sqrt{N}}$$

où $\overline{D}$ est la valeur de différence moyenne des paires pré- et postprandiales des glycémies ;
s est l'écart-type des mesures pré- et postprandiales ; et
$\Delta$ est la valeur de seuil prédéfinie,
N est le nombre de la pluralité de paires pré- et postprandiales ; et
$t_{\alpha, N-1}$ est une valeur critique d'une table statistique basée sur un niveau de signification et un degré de liberté.

4. Procédé selon la revendication 1, dans lequel dans la vérification comprend de calculer une quantité P et une valeur critique Q des formes respectives suivantes :

$$P = \frac{\overline{D} - \Delta}{s}$$

et

$$Q_{0,05,N} = 1,9182 \times N^{-0,5312}$$

où $\overline{D}$ est la valeur de différence moyenne des paires pré- et postprandiales des glycémies ;
s est l'écart-type des mesures pré- et postprandiales ; et
$\Delta$ est la valeur de seuil prédéfinie, et
N est le nombre de la pluralité de paires pré- et postprandiales.

5. Procédé selon l'une quelconque des revendications précédentes, comprenant en outre l'étape suivante :

procéder à une pluralité de mesures de glycémies pour transformer physiquement le glucose contenu dans un échantillon de fluide en un sous-produit enzymatique, et générer une quantité de médiateur réduit (par exemple, du ferrocyanure) proportionnelle à la glycémie de l'échantillon de fluide.

*FIG. 1A*

**FIG. 1B**

**FIG. 2A**

**FIG. 4**

**FIG. 2B**

**FIG. 3A**

**FIG. 3B**

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090240127 A1
- US 6179979 B
- US 6193873 B
- US 6284125 B
- US 6413410 B
- US 6475372 B
- US 6716577 B
- US 6749887 B
- US 6863801 B
- US 6890421 B
- US 7045046 B
- US 7291256 B
- US 7498132 B
- US 407173 A